# EUROPEAN PATENT SPECIFICATION

(11) **EP 3 755 387 B1**
(45) Date of publication and mention of the grant of the patent: **03.04.2024**
(21) Application number: 19757803.2
(22) Date of filing: 20.02.2019
(51) Int. Cl.: A61L 27/52, A61K 38/01, A61L 26/00, A61L 27/14, A61L 27/36, A61L 27/24, A61L 27/20, A61L 27/16, A61L 27/18

(54) **HIGH CONCENTRATION HYDROGELS AND RELATED METHODS**
HOCHKONZENTRIERTE HYDROGELE UND VERWANDTE VERFAHREN
HYDROGELS À HAUTE CONCENTRATION ET PROCÉDÉS ASSOCIÉS

(30) Priority: 20.02.2018 US 201862632690 P
(43) Date of publication of application: 30.12.2020
(73) Proprietor: ADVANCED AESTHETIC TECHNOLOGIES, INC., Brookline, MA 02445 (US)
(72) Inventor: PROVONCHEE, Richard, Cushing, Maine 04563 (US); BURTT, Richard, Brookline, Massachusetts 02445 (US)
(74) Representative: Pons
(86) International application number: PCT/US2019/018752
(87) International publication number: WO 2019/164931

(56) References cited:
- US-A1- 2005 046 069
- US-B1- 6 352 707
- US-B2- 8 883 184
- US-B2- 9 394 384
- BUCKLEY C T ET AL: "The effect of concentration, thermal history and cell seeding density on the initial mechanical properties of agarose hydrogels", JOURNAL OF THE MECHANICAL BEHAVIOR OF BIOMEDICAL MATERIALS, ELSEVIER, AMSTERDAM, NL, vol. 2, no. 5, 1 October 2009 (2009-10-01) , pages 512-521, XP026151519, ISSN: 1751-6161, DOI: 10.1016/J.JMBBM.2008.12.007 [retrieved on 2008-12-30]

## Description

### CROSS REFERENCE TO RELATED APPLICATION

This application claims priority from U.S. Provisional Application Serial No. 62/632,690, titled "Dehydrated Agarose Hydrogel Structures and Related Methods" filed February 20, 2018, the contents of which are incorporated by reference herein.

### BACKGROUND

US 9 394 384 B2 discloses a method of making tough hydrogels by dehydration and rehydration. The tough hydrogels can be used in a body to augment or replace any tissue such as cartilage, muscle, breast tissue, nucleus pulposus of the intervertebral disc, interpositional devices that generally serves as a cushion within a joint, etc. Tough hydrogels generally include polymer, polymer blends, or copolymers of polyvinyl alcohol (PVA), polyvinyl pyrrolidone (PVP), polyethylene oxide (PEO), polyacrylamide (PAAM), polyacrylic acid (PAA), alginates, polysaccharides, polyoxyethylene-polyoxypropylene co-polymers, poly-N-alkylacrylamides, poly-N-isopropyl acrylamide (PNIAAm), chondroitin sulfate, dextran sulfate, dermatin sulfate, or combinations thereof.

Hydrogels can be used for in-vivo applications, including filling and bulking, drug delivery, and scaffold generation. Agarose hydrogels are particularly promising for in-vivo applications.

### SUMMARY

It has been found that some applications benefit particularly when an agarose hydrogel is dehydrated and thereafter partially or fully rehydrated to form a high concentration hydrogel comprising agarose in a weight percent of at least 10%. Specific applications for dehydrated and rehydrated high concentration hydrogels containing agarose include but are not limited to use in or on a mammalian body. Example dehydrated and partially or fully rehydrated hydrogel structures are disclosed herein. As will be understood upon consideration of the subject disclosure, the disclosed hydrogel structures may be used for dermal filling, non-surgical lifting, cartilage augmentation, bone augmentation, orthopedic applications (such as cushioning between bones), non-surgical augmentation (e.g., for breasts, buttocks, or other anatomical features), cartilage replacement, guided nerve regeneration, tissue scaffolding, bone scaffolding, bulking, drug delivery, surgical mesh, viscosupplementation, and other different or related applications. The disclosed high concentration hydrogels may exhibit longer persistence in the body and also may, in some cases, be firmer and more flexible than other hydrogels.
Then, in a first aspect, the present invention refers to a method of forming a high concentration hydrogel as described in claim 1 and dependent claims 2 to 11 (the method of the present invention).
A second aspect of the invention refers to a high concentration hydrogel as described in claim 12 and a final aspect of the invention refer to a high concentration hydrogel for uses according to claims 13 to 16.

### BRIEF DESCRIPTION OF THE DRAWINGS

FIG. 1 is a flow diagram illustrating an exemplary process of forming a high concentration hydrogel, in accordance with some embodiments of the subject disclosure.
FIG. 2 is a photograph showing example dehydrated and subsequently rehydrated hydrogels having varying starting agarose compositions and drying conditions, in accordance with some embodiments of the subject disclosure.
FIG. 3 is a photograph showing example dehydrated hydrogels having varying starting agarose compositions, in accordance with some embodiments of the subject disclosure.
FIG. 4 is a photograph showing the example dehydrated hydrogels of FIG. 3 after rehydration, in accordance with some embodiments of the subject disclosure.
FIG. 5 is a photograph showing the example rehydrated hydrogels of FIG. 4 after having been stretched and flattened, in accordance with some embodiments of the subject disclosure.
FIGS. 6A-6F are photographs of an exemplary dehydrated hydrogel during a rehydration process, in accordance with some embodiments of the subject disclosure.

### DETAILED DESCRIPTION

Methods and techniques for producing high concentration hydrogels comprising agarose in a weight percent of at least 10% using various dehydration and rehydration techniques are provided herein. The disclosed dehydrated hydrogels (also referred to at times as [dehydrated] hydrogel structures) may be used for numerous applications, including in or on a mammalian body. For example, in some embodiments, the disclosed hydrogel structures may be used for dermal filling, non-surgical lifting, cartilage augmentation, bone augmentation, non-surgical augmentation (e.g., for breasts, buttocks, or other anatomical features), cartilage replacement, guided nerve regeneration, tissue scaffolding, bone scaffolding, bulking, drug delivery, surgical mesh, viscosupplementation, and other different or related applications. As described herein, the resulting high concentration hydrogels formed by a dehydration process followed by a rehydration process may be significantly more robust and flexible than the starting gel (prior to dehydration).

As used herein, the term "hydrogel" or simply "gel," refers to a hydrophilic network of polymer. Hydrogels are highly absorbent and, in some cases, are able to contain more than 90% water by weight. Any suitable type of hydrogel that comprises, consists of, or consists essentially of agarose may be used in the method of the present invention. In some embodiments, the hydrogel may also comprise: methylcellulose, hyaluronic acid, silicone, polyacrylamides, polymacon, alginate, chitosan, collagen, and/or polyethylene oxide. The starting concentration of agarose in hydrogels may be at least 0.1%, 1%, 2%, 3%, 4%, 5%, 6%, 7%, 8%, 9%, 10%, or more by weight. In some embodiments, some or all of the agarose used may be derivatized or ligand coupled or chemically cross-linked or irradiated or a combination thereof. In some such embodiments, any type of agarose can be used (e.g., low electroendosmosis (LE) agarose). If desired, other compounds or additives may also be included in the hydrogel.

Hydrogel additives may be therapeutic or used to improve the physical characteristics of the rehydrated or not yet rehydrated gel, or both. Hyaluronic acid (HA) can be both therapeutic and act as a humectant to make the dehydrated gel somewhat more flexible and/or soft. Other humectants known in the art, for example, sorbitol and glycerin may also be used in the disclosed hydrogels. In some embodiments, HA or a pharmaceutically acceptable salt thereof may be present in the hydrogel (either prior to or after dehydration) in a weight percent of between 0.1 and 4% or higher. In these and other embodiments, one or more enzymes, proteins, and/or amino acids may also be included in the hydrogel. In certain embodiments, enzymes to hydrolyze or break one or more bonds of the hydrogel or to liquify the hydrogel from a gelled state may be present. For example, in some embodiments, the enzyme hyaluronidase (Hylenex) may be included. In these and other embodiments, the protein resilin (for example, in amounts between 0.01 and 0.1% by weight prior to or after dehydration) may be included along with, in some cases, isoleucine, leucine, glycine, alanine, valine, lysine, and/or serine. In some embodiments, a crosslinker may be added to the hydrogel, either before or after dehydration.

The term "water" as used herein refers to liquid H₂O in essentially pure form as well as mixed with other fluids and/or solids or in bodily fluids (e.g., a saline solution is included within the definition of the term "water").

The term "original volume' as used herein refers to the volume of a hydrogel at the time of gel formation.

The term "hydrogel structure" as used herein, refers to a hydrogel precursor solution that has been cross-linked.

The term "fully rehydrated" as used herein refers to a hydrogel that has reached a hydration state that is equivalent to an equilibrium hydration state that would be reached in the presence of excess water. The term 'rehydtrate' as used herein refers to increasing the water content of a previously dehydrated hydrogel. Depending on the type of hydrogel and the extent of its dehydration, a dehydrated hydrogel may be fully rehydrated to the point of reaching its original volume. However, in some cases, after dehydration, the hydrogel may not be capable of reaching its original volume. The rehydrated hydrogel of the method of the present invention have a volume that is at least 30%, 40%, 50%, 60%, 70%, 80%, or 90% less than the original volume of the hydrogel.

The term "dehydrate" as used herein refers to methods of reducing the water content of a hydrogel.

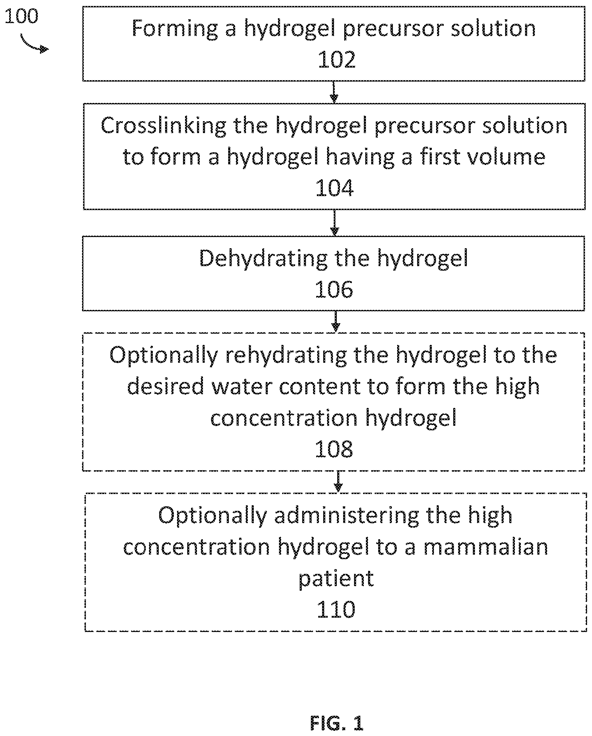

FIG. 1 is a flow chart illustrating an example method 100 of forming a high concentration hydrogel, in accordance with some embodiments of the subject disclosure. As shown in FIG. 1, method 100 includes forming a hydrogel precursor solution (Block 102). Forming a hydrogel precursor solution may be accomplished using any suitable technique or combination of techniques. The hydrogel precursor solution includes one or more hydrogels present in a solvent. The hydrogel precursor solution of the method of the present invention includes agarose dissolved in water or a non-aqueous solvent. The hydrogel solution may include hyaluronic acid. In embodiments, the weight percent of agarose in the hydrogel may be controlled, for example, by introducing a predetermined amount of water to a specific quantity of agarose.

In some embodiments, agarose may be present in a weight percent of between 0.1% and 10% or higher in the hydrogel precursor solution. Hyaluronic acid may be present in a weight percent of between 0.1% and 10% in the hydrogel precursor solution. In embodiments in which the hydrogel precursor solution contains both agarose and hyaluronic acid, agarose may be present in a greater weight percent than hyaluronic acid. In select embodiments, the concentration of agarose in the hydrogel precursor solution may be more than double the concentration of hyaluronic acid in the hydrogel precursor solution. It will be appreciated that the concentration of hydrogel(s) present in the hydrogel precursor solution may be selected based on end use or desired application. In some embodiments in which hyaluronic acid is present, the hyaluronic acid may be dissolved or may be present as gel particles. Additionally, if present, the hyaluronic acid in the hydrogel precursor solution may be cross-linked or not cross-linked. Since the viscosity of the hydrogel precursor solution increases as hydrogel concentration increases, in some embodiments, lower molecular weight hydrogels may be used for ease of processing. Numerous configurations and variations are possible and contemplated herein.

Method 100 of FIG. 1 continues with crosslinking the hydrogel precursor solution to form a hydrogel having a first volume (Block 104). Depending on the hydrogel solution, crosslinking may be accomplished, for example, by thermal or chemical or irradiation means or a combination thereof. Some methods of crosslinking hydrogel precursor solutions are known in the art. For example, some methods of thermally crosslinking a hydrogel precursor solution of agarose entail cooling a hydrogel precursor solution of agarose to below its gelling temperature. In some embodiments, a method of crosslinking a hydrogel precursor solution of agarose using a combination of thermal and chemical crosslinking entails thermally crosslinking the agarose solution by lowering the temperature of the solution to below the gelling temperature of the agarose and then exposing the thermally crosslinked agarose to epichlorohydrin, thereby chemically crosslinking the agarose. Numerous variations are possible and contemplated herein.

The hydrogel formed by crosslinking the hydrogel precursor solution may have a particular shape or structure, usually determined by the shape of the hydrogel precursor solution as it is crosslinked. As used herein, the term "hydrogel structure" refers to a particular shape of a hydrogel imparted by casting the hydrogel precursor solution during crosslinking. Often, casting occurs by forcing the hydrogel precursor solution (typically at an elevated temperature) into a desired shape as the hydrogel precursor solution is cooled. As the hydrogel solution cools, the hydrogel crosslinks and retains the shape in which it was cast. Casting may be accomplished by any suitable technique, including extruding a hydrogel precursor solution through a die having a desired cross-section, introducing droplets of hydrogel precursor solution into a non-aqueous (cooled) solvent, exposing droplets of hydrogel precursor solution to cool air, and other methods. In some embodiments, a hydrogel precursor solution may be cast by pouring a hydrogel precursor solution into a mold and allowing the hydrogel to crosslink in a quiescent state. In some such embodiments, the hydrogel will usually retain the shape of the mold when it is removed from the mold. Of course, a weak hydrogel structure may slump or deform when removed from the support provided by the mold.

The hydrogel may be formed into any desired structure or shape, depending on the intended use of the dehydrated hydrogel product. For example, in some embodiments, the hydrogel structure may be a bead, rod, thread, barbed thread, tube, ribbon, flat sheet, ordered or semi-ordered mesh, web, monolithic mass, cube, star, or other shape or structure. In some embodiments, a hydrogel structure may be formed with dimensions (e.g., volume, length, width, diameter, height, etc.) larger than the dehydrated hydrogel structure formed after water has been removed from the hydrogel structure. In some cases, the hydrogel structure may have dimensions at least 10%, 30%, 50%, 75%, or 90% greater than desired dimensions of the resulting dehydrated hydrogel structure. In some embodiments, the hydrogel structure may be reduced in size by cutting or grinding or cleaving or the like. This size reduction may take place before or after dehydration.

Method 100 continues with dehydrating the hydrogel (106). In particular, the hydrogel is dehydrated to have a water content that is less than a water content desired in the high concentration hydrogel ultimately produced in method 100. Any suitable technique may be used to dehydrate the hydrogel. For example, in some embodiments, the hydrogel may be dehydrated by evaporation, exposure to pressure, blotting, freeze/thaw processing, and/or contact with a dehydrating substance. In some embodiments, the hydrogel may be partially or fully dehydrated. For example, in some embodiments, the hydrogel may be dehydrated to have a water content less than 60%, less than 30%, or less than 10%. The extent of dehydration may depend on desired properties of the resulting product. Exemplary dehydration techniques are each described below in detail in the following paragraphs.

In embodiments in which the hydrogel is dehydrated using evaporation, evaporation may occur at ambient temperature and pressure, reduced pressure, increased temperature (for example, above 30°C, above 40°C, above 50°C, above 60°C, or between 30°C and 70°C, in some embodiments), decreased temperature (for example, at a temperature between 0°C and 25°C), increased air flow, and combinations thereof.

In embodiments in which the hydrogel is dehydrated using the application of pressure, water may be squeezed out of the hydrogel as the gel is pressed between plates or porous surfaces. Hydrogels that have been partially dewatered by pressing, may, in some cases, re-imbibe excess water and swell to some extent. The amount of swelling may be dependent on the amount of pressing force applied. In some embodiments, excess pressing may damage the hydrogel structure, thereby inhibiting re-swelling with water. For instance, an 1/8" rod of 1.5% agarose hydrogel will re-swell almost completely if pressed gently to express water and deform slightly. The same hydrogel rod will not re-swell, or will re-swell only slightly, if pressed aggressively to the point of flattening.

In embodiments in which water is drawn out of the hydrogel with blotting, a blotting agent, such as blotting paper or other water-absorbent structure may be used. In some cases, a hydrogel dehydrated by blotting may be capable of re-absorbing water.

In embodiments in which a freeze/thaw cycle is used to dehydrate a hydrogel, the internal structure of the hydrogel may collapse during freezing, causing the hydrogel to express a significant amount of water and possibly inhibiting or precluding the dehydrated hydrogel from re-imbibing water after dehydration has occurred.

In embodiments in which a hydrogel is dehydrated by contacting a dehydrating substance, a dehydrating liquid, such as a water-miscible hydrophilic liquid (e.g., acetone or isopropyl alcohol) may be used. In some such embodiments, the water may be diluted with the hydrophilic liquid and the water/hydrophilic liquid may then be evaporated or otherwise removed from the hydrogel. Numerous other methods of dehydrating a hydrogel structure are also contemplated.

In some embodiments, one or more than one technique may be used to dehydrate a hydrogel. Also, in some embodiments, the hydrogel may be partially or fully dehydrated. For example, in some cases, at least 5%, 10%, 20%, 25%, 30%, 40%, 50%, 60%, 70%, 75%, 80% or more water (as determined by weight percent) may be removed from the hydrogel during dehydration. As will be understood upon consideration of the subject disclosure, partially or fully dehydrating the hydrogel forms a dehydrated hydrogel.

Method 100 continues with optionally rehydrating the hydrogel to the desired water content to form the high concentration hydrogel (Block 108) (only the method comprising the rehydrating step is within the scope of the present invention). - The volume of the high concentration hydrogel may be at least 30%, 40%, 50%, 60%, 70%, or 80% less than the original volume of the hydrogel.

Rehydrating may be accomplished by exposing the dehydrated hydrogel structure to some amount of water and, in some cases, an excess supply of water. In some embodiments, the dehydrated hydrogel may be submerged in water to rehydrate the dehydrated hydrogel. In some embodiments, the dehydrated hydrogel may absorb essentially the same amount of water as was lost during dehydration or, in some cases, the dehydrated hydrogel may partially rehydrate by absorbing less water than was lost during dehydration.

In some embodiments, one or more materials may be added to the dehydrated hydrogel during rehydration. For example, in some embodiments, hyaluronic acid may be introduced during rehydration of the dehydrated hydrogel. In some such embodiments, the hyaluronic acid introduced during rehydration may or may not be cross-linked. If desired, the resulting rehydrated hydrogel may be cross-linked after rehydration.

In some cases, a partially rehydrated hydrogel may provide an increase in hydrogel (e.g., agarose) concentration. While agarose is reasonably easily workable in concentrations ≤4%, at higher concentrations, the viscosity of the solution becomes an issue and at concentrations above 6%, viscosity can become a significant issue. By forming a hydrogel at a workable concentration, dehydrating it and then partially rehydrating it, a material with a significantly higher hydrogel concentration can be produced that would be very difficult to work with in the usual way (i.e., without dehydration and rehydration). The high concentration hydrogel has an agarose concentration of at least 30% by weight. the high concentration hydrogel can include both agarose and hyaluronic acid. In select embodiments, the high concentration hydrogel may be free from sugars and other polysaccharides, if appropriate for the desired end use or application.

In some cases, the degree of dehydration may have a significant effect on the percent the dehydrated hydrogel may be rehydrated. That is, a hydrogel that is partially dehydrated may return to its original volume upon exposure to excess water. However, a hydrogel that is essentially fully dehydrated (for example, 90% or more water removed) may only return to 25% or less of its original volume during rehydration.

Except for hydrogels dehydrated by freeze/thaw methods, partially dehydrated hydrogels may gain volume up to original volume when exposed to excess water. The extent of volume regained during rehydration may depend on a number of factors. As previously discussed, the degree of dehydration may affect the percent of rehydration permitted. Additionally, the percent of agarose or other hydrogel(s) present may also affect the percent rehydration allowed by the hydrogel. According to the present invention, the hydrogel is rehydrated to the desired water content to form the high concentration hydrogel, wherein the high concentration hydrogel has a second volume that is at least 30% less than the first volume.

In some cases, the rate at which a dehydrated hydrogel is rehydrated depends, to some extent, on the temperature of the hydrogel during rehydration. For example, in some embodiments, the rate of rehydration can be increased by increasing the temperature at which the water is introduced to the dehydrated hydrogel. In some embodiments, rehydration is carried out at a temperature of at least 35°C, 40°C, 45°C, 50°C, 55°C, or at least 60°C. As will be appreciated, rehydration of the hydrogel can be carried out either in vitro or, at least partially, in vivo (with at least some rehydrating fluid being supplied by the body).

In select embodiments, the high concentration hydrogel is used to be administrated to a mammalian patient (Block 110). In some embodiments, the patient is a human. The high concentration hydrogel may be administered by any suitable technique, including via injection or topically. In select embodiments, the high concentration hydrogel is administered for one or more of the following purposes: wound care, cartilage augmentation, cartilage replacement, dermal filling, non-surgical lifting, bone augmentation, non-surgical augmentation, guided nerve regeneration, tissue scaffolding, bone scaffolding, bulking, drug delivery, surgical mesh, and viscosupplementation. If needed, the high concentration hydrogel may be sterilized prior to being administered to a mammalian patient.

A particularly interesting feature of dehydrated and subsequently rehydrated hydrogels is the change in brittleness and strength exhibited in the hydrogels that endure dehydration and rehydration. For instance, LE agarose is considered a brittle hydrogel. A 1% or 4% LE hydrogel rod will crack and break if it is bent into a tight 'u' shape. However, dehydrated and subsequently rehydrated specimens of LE agarose hydrogel are much more flexible and will not break when bent tightly. These dehydrated and subsequently rehydrated hydrogels are also tougher. Moreover, the increase in flexibility and toughness for dehydrated and rehydrated hydrogels does not appear to be a result solely of a higher concentration of hydrogel present in the rehydrated specimens but also a result of the dehydration and subsequent rehydration itself. It should also be noted that hydrogels that have been dehydrated and not subsequently rehydrated are surprisingly tough.

In some cases, water used to rehydrate the dehydrated hydrogel structure may have a salt content that is the same as or different from the salt concentration of the water used to form the hydrogel solution. Salt concentration of rehydrating water may, in some cases, affect the extent of rehydration. For example, water with less salt content or no salt present may rehydrate a dehydrated hydrogel structure to a greater extent than water with a higher salt concentration.

Although method 100 shown in FIG. 1 illustrates some features that may be used to form a high concentration hydrogel, numerous other possibilities are contemplated herein. For example, in select embodiments, various materials, such as dehydrated or partially rehydrated agarose hydrogel may be added during crosslinking of the hydrogel precursor solution. Additionally, in some cases, one or more therapeutic agents may be incorporated into the dehydrated hydrogel. In some such embodiments, the one or more therapeutic agents may be added before or after dehydration. As will be appreciated, the dehydrated (or subsequently rehydrated) hydrogel can be used in its natural shape or it may be cut, ground up, perforated, or otherwise altered prior to use.

In some aspect of the present disclosure not within the scope of the present invention, rehydration of the dehydrated hydrogel is optional, and in some cases, dehydrated hydrogels may be packaged and shipped while dehydrated, partially rehydrated (some water present, but excess water not present, and additional rehydration possible), or fully rehydrated (excess water present and further rehydration not possible). Similarly, dehydrated hydrogels may be used when dehydrated, partially rehydrated, or fully rehydrated. For example, in some particular embodiments, dehydrated hydrogels may be applied to (e.g., either in or on) a mammalian body while in a dehydrated state. In some such embodiments, most rehydration of the gel will take place at the target site using water provided by the target site or supplied to the target site or mixed with the hydrogel particles before application to the target site. A non-rehydrated or partially rehydrated form of dehydrated hydrogels may be useful when it is desirable to have the injected agarose increase in volume once it has reached the target site. This is particularly useful when the desired entry method to the target site precludes or makes difficult the delivery of the desired sized particles. It is also useful if it is desired to 'pack' the target site. As used herein, the term 'pack' means, for example, completely filling and providing intimate contact with the surface of the target site or even enlarging the target site somewhat through the swelling of the agarose or other hydrogels present.

In some aspect of the present disclosure not within the scope of the present invention, dehydrated hydrogels may also have a physical form that better lends it to certain application techniques than rehydrated structures. For example, delivery of threads may be easier if they have a stiffness that is somewhat greater than the stiffness of the rehydrated gel. As will be understood upon consideration of the subject disclosure, there may be cases in which the dehydrated hydrogel will start to rehydrate during or before its reaching the target site. As an example, if dehydrated particles are to be injected and the intent is to have them swell once at the site of injection, they may start swelling/rehydrating when mixed with an aqueous carrier liquid used for injection.

The disclosed high concentration hydrogels (in fully dehydrated form, partially rehydrated form, and fully rehydrated form, being only the partially or fully rehydrated hydrogels the ones within the scope of the present invention) may be used for any appropriate application in or on a mammalian body. For example, in some embodiments, the disclosed methods may be used to form a dehydrated hydrogel in the form of a thread (e.g., a short thread having a low length to diameter ratio or a continuous thread having a high length to diameter ratio). For example, in some cases, a short thread may have a length to diameter ratio within the range of 2:1 to 50:1 and a continuous thread may have a length to diameter ratio of at least 50:1. In some embodiments, the threads may have barbs or other devices that produce an increased drag as the thread is drawn through tissue or the like. This increased drag can be uni-directional or bi-directional. These embodiments could be useful, for example in non-surgical skin lifting treatments as exemplified by the Silhouette-Soft^{®} suspension suture product offered by Sinclair Pharma. The barbs or devices could be incorporated into or on the thread at any stage of its formation. For example, in some embodiments, barbs may be cast into the hydrogel structure and maintain their basic shape through dehydration. For example, in some embodiments, barbs could be formed in the hydrogel structure before or after dehydration, for example, by cutting with a blade or a laser or water jet. In some embodiments, these barbs or devices could be separate pieces added to the thread before or after dehydration. In some example embodiments, the threads may be linear or non-linear. For example, in some embodiments, the threads may be crimped or wound into a spiral shape prior to use to increase springiness. In some example embodiments, dehydrated hydrogel thread structures may be wound onto a bobbin for efficient storage, shipping, and application. Threads formed of dehydrated hydrogels can be woven, knitted or non-woven into a mat or mesh before or after dehydration or rehydration and have application, for example, in wound care or as surgical mesh. The dehydrated hydrogel thread structure may have any desired diameter and, in some cases, may have an agarose concentration of up to 30% or 40%. In some particular embodiments, the disclosed dehydrated hydrogel thread structures may include one or more additives, such as a humectant or another material to impart a desired level of softness and flexibility to the thread. In these and other embodiments, the threads may include one or more therapeutic agents. The threads may or may not be cross-linked, depending on intended end use.

Upon injection into a mammalian body, a thread of dehydrated agarose hydrogel (whether fully dehydrated, partially rehydrated, or fully rehydrated) may tend to bunch at some points forming a nest-like or web-like structure. This nest-like or web-like structure may provide springiness to the injected material and may also diminish dislocation of the injected material in the body. The size and stiffness of the dehydrated hydrogel thread structures described herein may have an impact on properties of the nest or web structure formed by the threads. For example, soft thin threads may pack together with little or no space between threads, whereas firmer threads and thicker threads may produce a more open structure. The method and technique of injection may also have an impact on size, shape and feel of the resulting injected material. For example, in embodiments in which the disclosed dehydrated hydrogel thread structures are injected with another material, such as a fractured agarose hydrogel, the threads may provide additional support and structure for the fractured hydrogel, leading to more robust and springy injected material. Numerous variations are possible and contemplated. A few experimental examples are described below but are not intended to limit the scope of the subject disclosure.

### Experimental Examples

A 3% agarose gel rod having a diameter of approximately 4.4mm was dehydrated by warm air evaporation. The resultant material was clear, flexible and robust and had an agarose concentration of greater than 23%. When fully rehydrated in a 1% NaCL water solution, it was a rod of about 2mm with an agarose concentration of about 15%. There is no reason to believe that if the original gel rod had been cast at 500µm diameter, the resultant gel thread (fully rehydrated) would be four times the concentration and ∼250µm diameter and the dehydrated thread before rehydration ~100µm diameter. This experiment shows the utility and benefit of some embodiments of the subject disclosure. For example, agarose structures of high concentration with controlled size and shape are achievable.

In a separate experiment, a 4% agarose hydrogel rod was soaked in acetone for a long enough time to exchange the water in the gel with acetone. The size of the rod did not change but it turned whitish and was decidedly more brittle than the starting agarose. The rod dried quickly in a 40°C oven and was a firm white rod of reduced size. After an overnight soak in water, the rod had swelled somewhat more than a similar 4% agarose hydrogel rod that had been dried by evaporation at 40°C and soaked overnight. The rehydrated acetone dried rod was flexible but had a whitish core running down through the rod. It is possible that the water may not have yet displaced all of the acetone.

In a different experiment, the effect of agarose concentration and drying temperature on agarose rehydration was evaluated. In this experiment, two LE agarose gels, a 1% w/v and a 4% w/v in water were dried in a 40°C oven until clear (no haze evident). After drying, they were put into excess room temperature water overnight. Two other LE agarose gels, a 1% w/v and a 4% w/v in water were dried in a 60°C oven until clear (no haze evident). After drying, these gels were put into excess room temperature water overnight. The results with respect to rehydration are provided below in Table 1.

**Table 1 - The Effect of Agarose Concentration and Drying Temperature on Rehydration**

| | Sample title | Percent rehydrated | Final concentration of agarose |
|---|---|---|---|
| 1% agarose dried at 40°C, rehydrated overnight | Sample A | 8% | 13% |
| 4% agarose dried at 40°C, rehydrated overnight | Sample B | 24% | 17% |
| 1% agarose dried at 60°C, rehydrated overnight | Sample C | 5% | 20% |
| 4% agarose dried at 60°C, rehydrated overnight | Sample D | 13% | 30% |

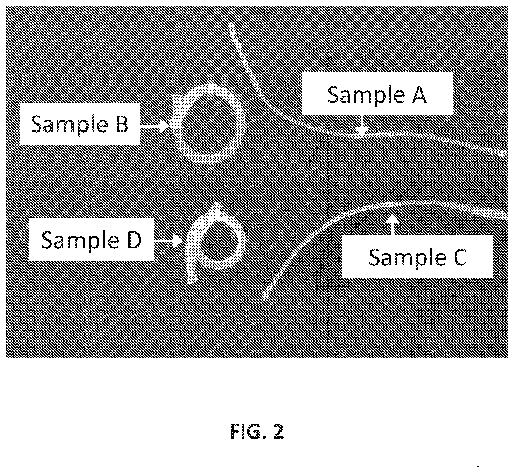

These results indicate that lower concentrations do not rehydrate as well as higher concentrations and that drying at a lower temperature enhances rehydration. FIG. 2 shows a photograph of the resulting sample gels shown in Table 1. In particular, in FIG. 2, sample A shows a 1% agarose gel that was dried at 40°C and rehydrated overnight, sample B shows a 4% agarose gel that was dried at 40°C and rehydrated overnight, sample C shows a 1% agarose gel that was dried at 60°C and rehydrated overnight, sample D shows a 4% agarose gel that was dried at 60°C and rehydrated overnight. It should be noted that the slight thickening of the ends of the 1% gels in the image is due to incomplete drying. It is also worth noting that these gels and most agarose gels that are dehydrated and then rehydrated are not only smaller in diameter than the original gel but are also shorter. It was surprising to find that these rehydrated gels could be easily stretched back to their original length without breaking and would stay at essentially that original length. A normal agarose gel that has not been dehydrated then rehydrated will simply break if stretched in this manner.

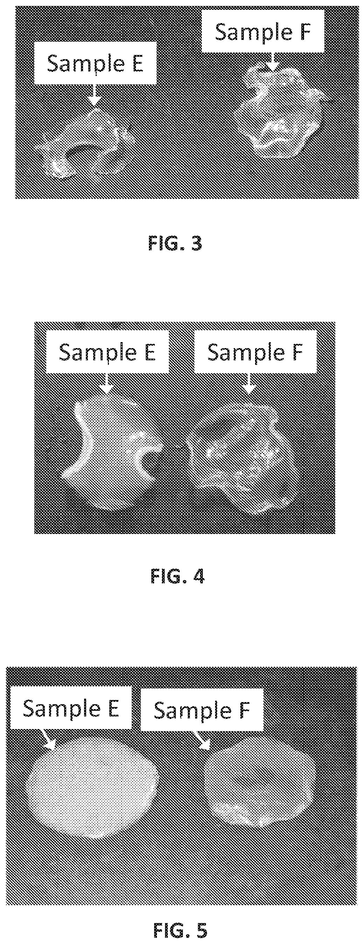

In another experiment, stretchy compliance of a rehydrated gel was studied. In this experiment, two gels were formed: a 1% LE and a 4% LE, each cast in a small cup giving gels approximately 60mm in diameter with a thickness of 3mm. These gels were dried on a screen in a 40°C drying oven for approximately 8 hours. FIG. 3 shows a photograph of the resulting dehydrated gels. In FIG. 3, Sample E is the 4% LE gel and Sample F is the 1% LE gel. The dehydrated gels were hard and miss-shaped. They were tough and not brittle. The gels were then soaked in room temperature water overnight. After the overnight soak, the gels had softened and relaxed significantly but were still miss-shaped. FIG. 4 shows the resulting rehydrated gels (Sample E and Sample F). The gels were then placed between two sheets of parchment paper and rolled gently with a 3" roller. FIG. 5 shows the resulting rolled gels (Sample E and Sample F). This rolling flattened and increased the diameter of the gels. This would not happen with an LE agarose gel that had not been dehydrated and rehydrated as a gel that had not been dehydrated would have fractured if rolled in this manner. After rehydration, the gels in this experiment were tough and rubbery - not at all like agarose gels that have not been dehydrated and then rehydrated. Gels with these properties could provide particular utility and benefit, for example, in cartilage replacement or augmentation or as cushioning in orthopedic applications.

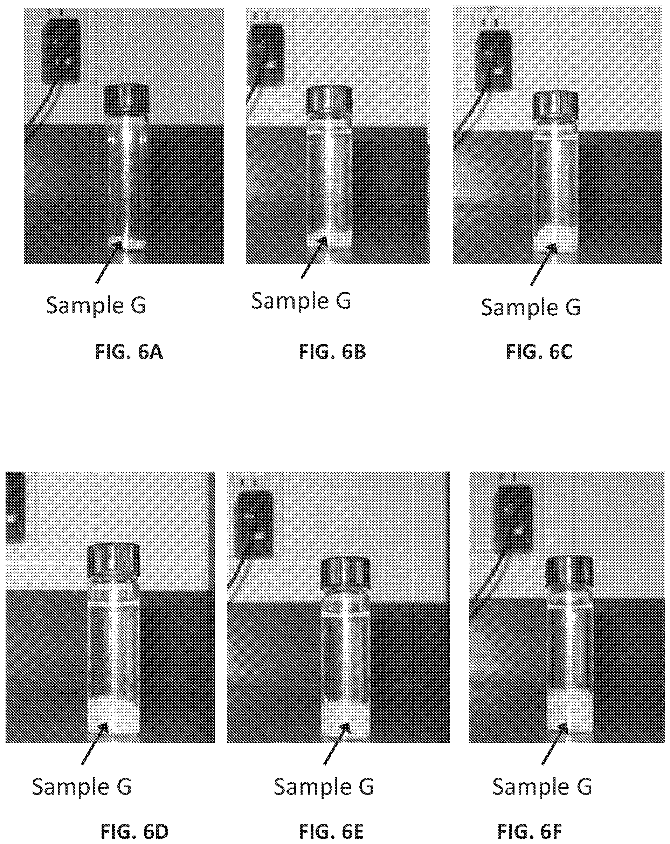

In another experiment, a pure agarose gel was dehydrated, chopped to form particulate dehydrated agarose, and exposed to water with a 1% salt (NaCl) content. FIGS. 6A-6F show the gel (Sample G) during a rehydration process. In particular, FIG. 6A illustrates Sample G at time 0, before addition of the 1% salt solution. FIG. 6B illustrates Sample G at time 2.5 minutes of exposure to the 1% salt solution. FIG. 6C illustrates Sample G at time 18 minutes, FIG. 6D illustrates Sample G at time 70 minutes, FIG. 6E illustrates Sample G at time 160 minutes, and FIG. 6F illustrates Sample G at time 220 minutes. These images show the extent particles of dehydrated agarose will swell when exposed to water with a salt content slightly higher than physiological saline.

## Claims

1. A method of forming a high concentration hydrogel, the method comprising:
forming a hydrogel precursor solution comprising one or more hydrogels in a solvent;
crosslinking the hydrogel precursor solution to form a hydrogel having a first volume;
dehydrating the hydrogel to have a water content less than a water content desired in the high concentration hydrogel; and
rehydrating the hydrogel to the desired water content to form the high concentration hydrogel, wherein the high concentration hydrogel has a second volume that is at least 30% less than the first volume, and wherein the high concentration hydrogel comprises agarose in a weight percent of at least 10%.

2. The method of claim 1, wherein the one or more hydrogels further comprises hydrogels selected from the group consisting of: methylcellulose, hyaluronic acid, silicone, polyacrylamides, polymacon, alginate, chitosan, collagen, and/or polyethylene oxide.

3. The method of claim 2, wherein the high concentration hydrogel comprises agarose in a weight percent of at least 20%.

4. The method of claim 1, wherein the high concentration hydrogel includes agarose and hyaluronic acid.

5. The method of claim 4, wherein the hyaluronic acid is introduced while forming the hydrogel precursor solution or during rehydration.

6. The method of claim 1, wherein the hydrogel is dehydrated by one or more of the following: evaporation, exposure to pressure, blotting, freeze/thaw processing, and contact with a dehydrating substance and the hydrogel is dehydrated to have a water content less than 60%, preferably less than 30%.

7. The method of claim 1, wherein the crosslinking is accomplished thermally or chemically, wherein the thermally crosslinking comprises cooling the hydrogel precursor solution below a gelling temperature of the hydrogel precursor solution.

8. The method of claim 1 further comprising casting the hydrogel precursor solution during crosslinking to impart a desired shape to the hydrogel to form a hydrogel structure.

9. The method of claim 8, wherein the hydrogel structure is selected from the group consisting of: beads, rods, threads, barbed threads, tubes, ribbons, flat sheets, ordered or semi-ordered meshes, webs, monolithic masses, cubes, and stars.

10. The method of claim 1, wherein dehydrating is carried out at a temperature of at least 40°C.

11. The method of claim 1, wherein rehydrating occurs in vitro.

12. A high concentration hydrogel formed using the method of claim 1.

13. The high concentration hydrogel formed using the method of claim 1 for use in wound care, cartilage augmentation, cartilage replacement, dermal filling, non-surgical lifting, bone augmentation, guided nerve regeneration, tissue scaffolding, bone scaffolding, drug delivery, surgical mesh and viscosupplementation.

14. The high concentration hydrogel formed using the method of claim 1 for use in one or more of the following: cartilage augmentation, dermal filling, non-surgical lifting, non-surgical augmentation, bulking and viscosupplementation, wherein the use comprises administering the high concentration hydrogel to a mammalian patient, preferably a human.

15. The high concentration hydrogel for use of claim 14, wherein the high concentration hydrogel is administered via injection or topically.

16. The high concentration hydrogel for use of claim 14, wherein the use further comprises sterilizing the high concentration hydrogel prior to administering to the mammalian patient.

## Patentansprüche

1. Verfahren zur Bildung eines hochkonzentrierten Hydrogels, wobei das Verfahren Folgendes umfasst:
Bilden einer Hydrogel-Vorläuferlösung, die ein oder mehrere Hydrogele in einem Lösemittel umfasst;
Vernetzen der Hydrogel-Vorläuferlösung, um ein Hydrogel zu bilden, das ein erstes Volumen aufweist;
Dehydratisieren des Hydrogels, sodass es einen Wassergehalt aufweist, der geringer ist als ein gewünschter Wassergehalt in dem hochkonzentrierten Hydrogel; und
Rehydratisieren des Hydrogels auf den gewünschten Wassergehalt, um das hochkonzentrierte Hydrogel zu bilden, wobei das hochkonzentrierte Hydrogel ein zweites Volumen aufweist, das mindestens 30 % geringer ist als das erste Volumen, und wobei das hochkonzentrierte Hydrogel Agarose in einem Gewichtsprozentanteil von mindestens 10 % umfasst.

2. Verfahren gemäß Anspruch 1, wobei das eine oder die mehreren Hydrogele ferner Hydrogele umfassen, die aus der Gruppe, bestehend aus Methylcellulose, Hyaluronsäure, Silikon, Polyacrylamiden, Polymacon, Alginat, Chitosan, Kollagen und/oder Polyethylenoxid, ausgewählt sind.

3. Verfahren gemäß Anspruch 2, wobei das hochkonzentrierte Hydrogel Agarose in einem Gewichtsprozentanteil von mindestens 20 % umfasst.

4. Verfahren gemäß Anspruch 1, wobei das hochkonzentrierte Hydrogel Agarose und Hyaluronsäure einschließt.

5. Verfahren gemäß Anspruch 4, wobei die Hyaluronsäure während der Bildung der Hydrogel-Vorläuferlösung oder während der Rehydratisierung eingebracht wird.

6. Verfahren gemäß Anspruch 1, wobei das Hydrogel durch einen oder mehrere der folgenden Schritte dehydratisiert wird: Verdampfen, Druckeinwirkung, Abtupfen, Einfrieren-Auftauen und Kontakt mit einer dehydratisierenden Substanz, und das Hydrogel so dehydratisiert wird, dass es einen Wassergehalt von weniger als 60 %, vorzugsweise weniger als 30 %, aufweist.

7. Verfahren gemäß Anspruch 1, wobei die Vernetzung thermisch oder chemisch erfolgt, wobei die thermische Vernetzung das Abkühlen der Hydrogel-Vorläuferlösung unter eine Geliertemperatur der Hydrogel-Vorläuferlösung umfasst.

8. Verfahren gemäß Anspruch 1, das ferner das Gießen der Hydrogel-Vorläuferlösung während der Vernetzung umfasst, um dem Hydrogel eine gewünschte Form zu verleihen und eine Hydrogelstruktur zu bilden.

9. Verfahren gemäß Anspruch 8, wobei die Hydrogelstruktur aus der Gruppe, bestehend aus Kügelchen, Stäben, Fäden, mit Widerhaken versehenen Fäden, Röhren, Bändern, flachen Lagen, geordneten oder halbgeordneten Maschen, Netzen, monolithischen Massen, Würfeln und Sternen, ausgewählt ist.

10. Verfahren gemäß Anspruch 1, wobei die Dehydratisierung bei einer Temperatur von mindestens 40 °C durchgeführt wird.

11. Verfahren gemäß Anspruch 1, wobei die Rehydratisierung *in vitro* erfolgt.

12. Hochkonzentriertes Hydrogel, das unter Verwendung des Verfahrens gemäß Anspruch 1 gebildet wird.

13. Hochkonzentriertes Hydrogel, das unter Verwendung des Verfahrens gemäß Anspruch 1 gebildet wird, zur Verwendung für folgende Zwecke: Wundversorgung, Knorpelaugmentation, Knorpelersatz, Hautfüllung, nichtchirurgisches Liften, Knochenaugmentation, gesteuerte Nervenregeneration, Gewebegerüst, Knochengerüst, Arzneimittelabgabe, chirurgisches Netz und Viskosupplementation.

14. Hochkonzentriertes Hydrogel, das unter Verwendung des Verfahrens gemäß Anspruch 1 hergestellt wird, zur Verwendung für einen oder mehrere der folgenden Zwecke: Knorpelaugmentation, Hautfüllung, nichtchirurgisches Liften, nichtchirurgische Augmentation, Volumenbildung und Viskosupplementation, wobei die Verwendung die Verabreichung des hochkonzentrierten Hydrogels an einen Säugetier-Patienten, vorzugsweise einen Menschen, umfasst.

15. Hochkonzentriertes Hydrogel zur Verwendung gemäß Anspruch 14, wobei das hochkonzentrierte Hydrogel durch Injektion oder topisch verabreicht wird.

16. Hochkonzentriertes Hydrogel zur Verwendung gemäß Anspruch 14, wobei die Verwendung ferner das Sterilisieren des hochkonzentrierten Hydrogels vor der Verabreichung an den Säugetier-Patienten umfasst.

## Revendications

1. Procédé de formation d'un hydrogel à haute concentration, le procédé comprenant :
la formation d'une solution précurseur d'hydrogel comprenant un ou plusieurs hydrogels dans un solvant ;
la réticulation de la solution précurseur d'hydrogel pour former un hydrogel ayant un premier volume ;
la déshydratation de l'hydrogel pour avoir une teneur en eau inférieure à une teneur en eau souhaitée dans l'hydrogel à haute concentration ; et
la réhydratation de l'hydrogel à la teneur en eau souhaitée pour former l'hydrogel à haute concentration, dans lequel l'hydrogel à haute concentration a un second volume qui est au moins 30 % inférieur au premier volume et dans lequel l'hydrogel à haute concentration comprend de l'agarose en un pourcentage en poids d'au moins 10 %.

2. Procédé selon la revendication 1, dans lequel le ou les hydrogels comprennent en outre des hydrogels choisis dans le groupe constitué par : de la méthylcellulose, de l'acide hyaluronique, de la silicone, des polyacrylamides, du polymacon, de l'alginate, du chitosane, du collagène et/ou du poly(oxyde d'éthylène).

3. Procédé selon la revendication 2, dans lequel l'hydrogel à haute concentration comprend de l'agarose en un pourcentage en poids d'au moins 20 %.

4. Procédé selon la revendication 1, dans lequel l'hydrogel à haute concentration renferme de l'agarose et de l'acide hyaluronique.

5. Procédé selon la revendication 4, dans lequel l'acide hyaluronique est introduit lors de la formation de la solution précurseur d'hydrogel ou pendant la réhydratation.

6. Procédé selon la revendication 1, dans lequel l'hydrogel est déshydraté par un ou plusieurs de ce qui suit : une évaporation, une exposition à de la pression, un tamponnement, un traitement de congélation/décongélation et un contact avec une substance déshydratante et l'hydrogel est déshydraté pour avoir une teneur en eau inférieure à 60 %, de préférence inférieure à 30 %.

7. Procédé selon la revendication 1, dans lequel la réticulation est réalisée thermiquement ou chimiquement, dans lequel la réticulation thermique comprend le refroidissement de la solution précurseur d'hydrogel au-dessous d'une température de gélification de la solution précurseur d'hydrogel.

8. Procédé selon la revendication 1 comprenant en outre le coulage de la solution précurseur d'hydrogel pendant la réticulation pour conférer une forme souhaitée à l'hydrogel pour former une structure en hydrogel.

9. Procédé selon la revendication 8, dans lequel la structure en hydrogel est choisie dans le groupe constitué par : des billes, des bâtonnets, des fils, des fils crantés, des tubes, des rubans, des feuilles plates, des grillages ordonnés ou semi-ordonnés, des toiles, des masses monolithiques, des cubes et des étoiles.

10. Procédé selon la revendication 1, dans lequel la déshydratation est effectuée à une température d'au moins 40 °C.

11. Procédé selon la revendication 1, dans lequel la réhydratation a lieu *in vitro.*

12. Hydrogel à haute concentration formé à l'aide du procédé selon la revendication 1.

13. Hydrogel à haute concentration formé à l'aide du procédé selon la revendication 1 destiné à être utilisé en soin de plaies, augmentation de cartilage, remplacement de cartilage, comblement cutané, lifting non chirurgical, augmentation osseuse, régénération nerveuse guidée, échafaudage tissulaire, échafaudage osseux, administration de médicaments, grillage chirurgical et viscosupplémentation.

14. Hydrogel à haute concentration formé à l'aide du procédé selon la revendication 1 destiné à être utilisé dans un ou plusieurs de ce qui suit : une augmentation de cartilage, un comblement cutané, un lifting non chirurgical, une augmentation non chirurgicale, un accroissement de volume et une viscosupplémentation, l'utilisation comprenant l'administration de l'hydrogel à haute concentration à un patient mammifère, de préférence un être humain.

15. Hydrogel à haute concentration destiné à être utilisé selon la revendication 14, l'hydrogel à haute concentration étant administré par le biais d'une injection ou par voie topique.

16. Hydrogel à haute concentration destiné à être utilisé selon la revendication 14, l'utilisation comprenant en outre la stérilisation de l'hydrogel à haute concentration avant l'administration au patient mammifère.
